# NEW EUROPEAN PATENT SPECIFICATION

(11) **EP 0 271 306 B2**
(45) Date of publication and mention of the opposition decision: **16.07.1997**
(45) Mention of the grant of the patent: 01.04.1992
(21) Application number: 87310749.4
(22) Date of filing: 07.12.1987
(51) Int. Cl.: A61K 9/06

(54) **Veterinary treatment**
Tiermedizinische Behandlung
Traitement vétérinaire

(30) Priority: 10.12.1986 GB 8629481
(43) Date of publication of application: 15.06.1988
(73) Proprietor: BEECHAM GROUP PLC, Brentford, Middlesex TW8 9EP (GB)
(72) Inventor: Dowrick, John Sidney, Tadworth Surrey, KT20 7NT (GB)
(74) Representative: Lederer, Franz, Dr.

(56) References cited:
- EP-A- 0 233 559
- GB-A- 661 970
- GB-A- 792 545
- GB-A- 1 312 918
- GB-A- 1 532 993
- AUFBEREITUNGS-TECHNIK, vol.12, no.9, September 1971, Verlag für Aufbereitung Schirmer & Zeh, Wiesbaden (DE); N.RINK et al, pp.562-572
- Novel drug delivery systems, ed. Y W Chien, Marcel Dekker Inc., 1982, p219-236, 304, 305
- A new absorption delaying vehicle for penicillin, Buckwater and Dickinson, J Maer Pharm Assoc., 47:661 (1985), p 472-474
- Kirk-Othmer's Encyclopedia of Chemical Technology. vol.21 3rd ed., 1983, p.134
- Proceedings of the Seminar on Mastitis Control 1975 held under the auspices of the International Dairy Federation on 7-11 April 1975, p.341-344

## Description

This invention relates to a veterinary composition for use in the treatment of mammary disorders in animals, especially bovine mastitis, and a process for its preparation.

It is known to use antibiotics and other medicaments in the treatment and prophylaxis of bovine mastitis. These antibacterial agents have previously been administered by the intramammary route and have therefore been used in a liquid or semi-liquid form such as a gel. In such formulations there is often little control over the rate of release of the active compound into the body of the animal. It is known that the efficiency of the treatment is partly dependent upon the duration of antibacterial activity and a prolonged release period is therefore desirable.

GB-A-1,312,918 describes a method of treating mammary disorders in non-human animals which comprises administering an antibacterial agent to the animal by the intramammary route during the animal's dry period, the antibacterial agent being administered in the form of sustained release beadlets.

GB-A-1,455,296 describes a method for the treatment or prophylaxis of mammary disorders in non-human animals which comprises administering an antibacterial agent to the animal by the intramammary route during the animal's dry period, the antibacterial agent being administered in the form of granules comprising the antibacterial agent in admixture with an insoluble polymeric binding agent.

GB-A-792,545 describes an antibiotic preparation in which antibiotic particles are coated with an aluminum salt of a fatty acid and dispersed in an oil to achieve a protracted effect.

GB-A-1,532,993 describes an injectable composition in which fine particles of amoxycillin trihydrate are coated with a dispersing agent so as to be dispersible in water.

GB-A-661,970 describes intramuscularly injectable suspensions of penicillins in gelled fatty oils in which the penicillin has a particle size of less than 50 micronmeter so as to yield prolonged therapeutic blood levels.

In the commercial product ORBENIN DC (Dry Cow) particles of the antibacterial agent benzathine cloxacillin are dispersed in a gelled oil.

It has now been discovered that the release period can be prolonged by the use of an antibacterial agent in the form of very finely divided particles, thus obviating the need for specially formulated beadlets, coatings, granules etc.

The present invention provides a process for the preparation of a veterinary composition for use in the treatment or prophylaxis of mammary disorders in non-human anmials by administration via the intramammary route during the animal's dry period, comprising an antibacterial agent in the form of uncoated particles, of which at least 65% by weight have a size in the range of 0-5 µm, at least 94% by weight have a size in the range 0-13 µm and less than 55% by weight are larger than 3 µm, suspended in a hydrophobic oily vehicle which comprises a mineral oil and a gelling agent, wherein the mixture obtained by mixing the antibacterial agent with the vehicle is subjected to bead-milling. The invention also provides a veterinary composition for use in the treatment or prophylaxis of mammary disorders in non-human animals by administration via the intramammary route during the animal's dry period, which composition comprises an antibacterial agent in the form of uncoated particles of which at least 65% by weight have a size in the range 0-5 µm, at least 94% by weight have a size in the range 0-13 µm, and less than 55% by weight are larger than 3 µm, as obtainable by bead-milling suspended in a hydrophobic oily vehicle which comprises a mineral oil and a gelling agent.

The invention is especially useful in the treatment or prophylaxis of bovine mastitis in which the antibacterial agent may be administered directly into the mammary gland via the streak canal.

The invention is primarily applicable in the dry-period therapy of bovine mastitis. It would clearly be undesirable if the composition continued to release appreciable quantities of antibacterial agent in the mammary gland after the beginning of lactation, since the antibacterial agent would then appear in the milk destined eventually for human consumption. The composition should therefore be formulated in such a way that either it is milked out at the beginning of lactation or alternatively has released substantially all the antibacterial agent before the beginning of lactation. This latter method of ensuring that appreciable quantities of active agent do not appear in the milk is preferred since generally it will be a routine matter to formulate the composition accordingly. Local regulations governing the maximum permissible quantity of active agent in the milk, the duration of the cow's dry period and local husbandry practice are all factors which can readily be taken into account when formulating the composition.

The vehicle is selected so as to be non-toxic, veterinary acceptable, compatible with the antibacterial agent, and of a viscosity to permit administration using a syringe at ambient temperature, whilst controlling the release characteristics of the finely divided drug particles.

Suitably the vehicle is a mineral oil base gelled with a gelling agent such a aluminum stearate, preferably liquid paraffin containing 0 to 5% by weight of aluminum (mono- or di-) stearate and from 0 to 2% by weight of stearic acid.

Thickening agents such as 12-hydroxystearin, beeswax, hydrogenated peanut or castor oil or soft or hard paraffin may also be used.

The antibacterial agent will normally represent 0.1 to 40%, more suitably 1 to 40% w/w of the veterinary composition. Particularly suitable ranges are 5 to 30% w/w.

The composition may also contain pain relieving agents, corticosteroids and the like. It may also be desirable to include an antioxidant such as butylated hydroxyanisole (Embanox - Trade Mark) in certain formulations.

Preferably the veterinary compositions for intramammary use are formulated as unit doses containing a therapeutically effective amount of benzathine cloxacillin. For use in the dry cow the preferred unit dose may contain 100 to 1000 mg more preferably 250 to 700 mg of benzathine cloxacillin. A typical dose is 500-600 mg.

A single dose of the composition will normally contain 1 to 20g of the formulated composition, preferably 2 to 10g. Typical formulations may contain 3 - 4g.

In the treatment of mastitis in dry cows a single dose applied to the infected quarter may be effective. However one or more further doses may be applied, depending on the length of the dry period, and one or more prophylactic doses may also be applied to the non-infected quarters.

For prophylactic treatment all quarters may be infused at drying off irrespective of whether there is any infection present.

The formulation would suitably be administered to the cow by means of an intramammary syringe, a tube or by other suitable packs which contain a unit dose of the formulation. Such a syringe is provided with a cannula nozzle for insertion into the teat to allow extrusion of the formulation directly into the mammary gland via the streak canal.

With the formulations of the present invention it is possible to maintain antibacterial levels in the udder for a longer period than can normally be achieved. The antibacterial agent is chosen from antibiotics and other compounds which are effective in the treatment of mammary disorders such as bovine mastitis, especially a penicillin or a non-toxic salt or ester thereof. Examples of suitable antibacterial agents are cloxacillin, especially benzathine cloxacillin, flucloxacillin, and its benzathine salt, amoxycillin, ampicillin, carbenicillin, dicloxacillin, methicillin, penicillin G especially the benzyl ester, temocillin, ticarcillin, neomycin especially the sulphate, streptomycin, novobiocin, tetracycline, chlortetracycline, oxytetracycline and salts thereof and mixtures of these compounds, e.g. benzathine flucloxacillin/ ampicillin trihydrate or benzathine diampicillin mixtures. The formulation may additionally contain a β-lactamase inhibitor such as potassium clavulanate.

The process may suitably be carried out as follows:-
(a) the oil is heated, the gelling or thickening agent is mixed in and the oil allowed to cool,
(b) the powdered active ingredient is mixed into the base with stirring,
(c) high shear mixing equipment is used to produce a fine homogenous dispersion, and
(d) the mixture is subjected to bead-milling.

The formulation is then packed in an appropriate form for administration, eg. syringe or tube.

The following Examples illustrate the invention.

### Example 1

| | |
|---|---|
| benzathine cloxacillin equivalent to cloxacillin | 83.3g |
| base | to 500 g |

The base had the following composition:

| | |
|---|---|
| aluminium stearate | 3.0% |
| stearic acid | 0.8% |
| liquid paraffin | to 100 % |

A gel of the mineral oil base was formed by dissolving the aluminium stearate and stearic acid in the heated liquid paraffin. After cooling the sterile milled benzathine cloxacillin was incorporated by high shear stirring. Typically 60-80% by weight of the particles of the benzathine cloxacillin used were larger than 3µm. After stirring this proportion increased to 65-95% by weight due to the formation of aggregates. The mixture was then subjected to bead milling by pumping through a high speed rotary bead mill under the following conditions:

| | | |
|---|---|---|
| *Model | 'Dyno-mill' | Model KDL Pilot |
| | Zirconium beads | 0.75-1.0 mm diameter |
| | Gap setting | 0.2 mm |
| | Rotor speed | 1910 rpm |

| | | |
|---|---|---|
| * Supplied by WAB, Basel, Switzerland | | |

The suspension was filled as 3.6g doses into intramammary syringes.

### Example 2

| | g | % |
|---|---|---|
| Ampicillin Trihydrate | 780 | 9.84 (8.3% as free acid) |
| Benzathine Cloxacillin | 1759 | 22.2 (16% as free acid) |
| Aluminum Stearate | 164 | 2.07 |
| Liquid Paraffin | 5221 | 65.88 |

A base was prepared by heating together and sterilising the aluminum stearate and liquid paraffin. When cool the sterile antibiotics were added and incorporated by high shear mixing. The mixture was then passed through a rotary bead mill under the following conditions:
- Equipment :: 'Dyno-Mill' Model KDL Pilot
- Zirconium beads :: 1.0-1.25mm
- Gap setting :: 0.3mm
- Feed rate :: 4.2 litres/hour
- Rotor speed :: 2400 RPM

Part of the finished suspension was filled as 3g doses into intramammary syringes.

## Claims (Claims for the following Contracting State(s): AT, BE, CH, DE, FR, GB, GR, IT, LI, LU, NL, SE)

1. A process for the preparation of a veterinary composition for use in the treatment or prophylaxis of mammary disorders in non-human anmials by administration via the intramammary route during the animal's dry period, comprising an antibacterial agent in the form of uncoated particles, of which at least 65% by weight have a size in the range of 0-5 µm, at least 94% by weight have a size in the range 0-13 µm and less than 55% by weight are larger than 3 µm, suspended in a hydrophobic oily vehicle which comprises a mineral oil and a gelling agent, wherein the mixture obtained by mixing the antibacterial agent with the vehicle is subjected to bead-milling.

2. Process according to claim 1 wherein the mineral oil is liquid paraffin.

3. Process according to one of the preceding claims wherein the antibacterial agent is a penicillin or a nontoxic salt or ester thereof.

4. Process according to one of the preceding claims wherein the antibacterial agent comprises benzathine cloxacillin and/or ampicillin.

5. Process according to claim 4 wherein the composition is a unit dose containing 600 mg of benzathine cloxacillin.

6. A veterinary composition for use in the treatment or prophylaxis of mammary disorders in non-human animals by administration via the intramammary route during the animal's dry period, which composition comprises an antibacterial agent in the form of uncoated particles of which at least 65% by weight have a size in the range 0-5 µm, at least 94% by weight have a size in the range 0-13 µm, and less than 55% by weight are larger than 3 µm, as obtainable by bead-milling suspended in a hydrophobic oily vehicle which comprises a mineral oil and a gelling agent.

## Claims (Claims for the following Contracting State(s): ES)

1. A process for the preparation of a veterinary composition for use in the treatment or prophylaxis of mammary disorders in non-human anmials by administration via the intramammary route during the animal's dry period, comprising an antibacterial agent in the form of uncoated particles, of which at least 65% by weight have a size in the range of 0-5 µm, at least 94% by weight have a size in the range 0-13 µm and less than 55% by weight are larger than 3 µm, suspended in a hydrophobic oily vehicle which comprises a mineral oil and a gelling agent, wherein the mixture obtained by mixing the antibacterial agent with the vehicle is subjected to bead-milling.

2. Process according to claim 1 wherein the mineral oil is liquid paraffin.

3. Process according to one of the preceding claims wherein the antibacterial agent is a penicillin or a nontoxic salt or ester thereof.

4. Process according to one of the preceding claims wherein the antibacterial agent comprises benzathine cloxacillin and/or ampicillin.

5. Process according to claim 4 wherein the composition is a unit dose containing 600 mg of benzathine cloxacillin.

## Patentansprüche (Patentansprüche für folgende(n) Vertragsstaat(en): AT, BE, CH, DE, FR, GB, GR, IT, LI, LU, NL, SE)

1. Verfahren zur Herstellung eines Veterinärarzneimittels zur Verwendung bei der Behandlung oder Prophylaxe von Eutererkrankungen bei Tieren durch Verabreichung über den intramammarischen Weg während der Trockenperiode des Tieres, umfassend ein antibakterielles Mittel in Form unbeschichteter Teilchen von denen mindestens 65 Gew.-% eine Größe im Bereich von 0 bis 5 µm, mindestens 94 Gew.-% eine Größe im Bereich von 0 bis 13 µm besitzen und weniger als 55 Gew.-% größer als 3 µm sind, suspendiert in einem hydrophoben öligen Bindemittel, umfassend ein Mineralöl und ein Gelierungsmittel, wobei das durch Mischen des antibakteriellen Mittels mit dem Bindemittel erhaltene Gemisch einem Kugelvermahlen unterworfen wird.

2. Verfahren nach Anspruch 1, wobei das Mineralöl flüssiges Paraffin ist.

3. Verfahren nach einem der vorstehenden Ansprüche, wobei das antibakterielle Mittel ein Penicillin oder ein nicht-toxisches Salz oder Ester davon ist.

4. Verfahren nach einem der vorstehenden Ansprüche, wobei das antibakterielle Mittel Benzathin-Cloxacillin und/oder Ampicillin umfaßt.

5. Verfahren nach Anspruch 4, wobei das Arzneimittel eine Einheitsdosis enthaltend 600 mg Benzathin-Cloxacillin ist.

6. Veterinärarzneimittel zur Verwendung bei der Behandlung oder Prophylaxe von Eutererkrankungen bei Tieren durch Verabreichung über den intramammarischen Weg während der Trockenperiode des Tieres, wobei das Mittel ein antibakterielles Mittel in Form von unbeschichteten Teilchen umfaßt, von denen mindestens 65 Gew.-% eine Größe im Bereich von 0 bis 5 µm, mindestens 94 Gew.-% eine Größe im Bereich von 0 bis 13 µm besitzen und weniger als 55 Gew.-% größer als 3 µm sind, wie durch Kugelvermahlen erhältlich, suspendiert in einem hydrophoben öligen Bindemittel, das ein Mineralöl und ein Gelierungsmittel umfaßt.

## Patentansprüche (Patentansprüche für folgende(n) Vertragsstaat(en): ES)

1. Verfahren zur Herstellung eines Veterinärarzneimittels zur Verwendung bei der Behandlung oder Prophylaxe von Eutererkrankungen bei Tieren durch Verabreichung über den intramammarischen Weg während der Trockenperiode des Tieres, umfassend ein antibakterielles Mittel in Form unbeschichteter Teilchen, von denen mindestens 65 Gew.-% eine Größe im Bereich von 0 bis 5 µm, mindestens 94 Gew.-% eine Größe im Bereich von 0 bis 13 µm besitzen und weniger als 55 Gew.-% größer als 3 µm sind, suspendiert in einem hydrophoben öligen Bindemittel, umfassend ein Mineralöl und ein Gelierungsmittel, wobei das durch Mischen des antibakteriellen Mittels mit dem Bindemittel erhaltene Gemisch einem Kugelvermahlen unterworfen wird.

2. Verfahren nach Anspruch 1, wobei das Mineralöl flüssiges Paraffin ist.

3. Verfahren nach einem der vorstehenden Ansprüche, wobei das antibakterielle Mittel ein Penicillin oder ein nicht-toxisches Salz oder Ester davon ist.

4. Verfahren nach einem der vorstehenden Ansprüche, wobei das antibakterielle Mittel Benzathin-Cloxacillin und/oder Ampicillin umfaßt.

5. Verfahren nach Anspruch 4, wobei das Mittel eine Einheitsdosis enthaltend 600 mg Benzathin-Cloxacillin ist.

## Revendications (Revendications pour l'(les) Etat(s) contractant(s) suivant(s): AT, BE, CH, DE, FR, GB, GR, IT, LI, LU, NL, SE)

1. procédé pour la préparation d'une composition vétérinaire utile dans le traitement ou la prophylaxie de troubles mammaires chez les animaux non-humains par administration par voie intramammaire pendant la période sèche de l'animal, comprenant un agent antibactérien sous la forme de particules non-enduites dont au moins 65% en poids ont une dimension comprise dans la gamme de 0 à 5 µm, au moins 94% en poids ont une dimension comprise dans la gamme de 0 à 13 µm et moins de 55% en poids ont une dimension supérieure à 3 µm, en suspension dans un véhicule huileux hydrophobe qui comprend une huile minérale et un agent gélifiant, dans lequel le mélange obtenu par mélange de l'agent antibactérien avec le véhicule est traité dans un broyeur à billes.

2. Procédé suivant la revendication 1, dans lequel l'huile minérale est une paraffine liquide.

3. Procédé suivant la revendication 1 ou la revendication 2, dans lequel l'agent antibactérien est une pénicilline ou un sel ou un ester non toxique de celle-ci.

4. Procédé suivant l'une quelconque des revendications précédentes, dans lequel l'agent antibactérien comprend de la benzathine cloxacilline et/ou de l'ampicilline.

5. Procédé suivant la revendication 4, dans lequel la composition vétérinaire est une dose unitaire contenant 600 mg de benzathine cloxacilline.

6. Composition vétérinaire utile dans le traitement ou la prophylaxie de troubles mammaires chez des animaux non-humains par administration par voie intramammaire pendant la période sèche de l'animal, laquelle composition comprend un agent antibactérien sous la forme de particules non-enduites dont au moins 65% en poids ont une dimension dans la gamme de 0,5 µm, au moins 94% en poids ont une dimension dans la gamme de 0 à 13 µm et moins de 55% en poids ont une dimension supérieure à 3 µm, conune celles qui peuvent être obtenues par traitement dans un broyeur à billes, en suspension dans un véhicule huileux hydrophobe qui comprend une huile minérale et un agent gélifiant.

## Revendications (Revendications pour l'(les) Etat(s) contractant(s) suivant(s): ES)

1. procédé pour la préparation d'une composition vétérinaire utile dans le traitement ou la prophylaxie de troubles mammaires chez les animaux non-humains par administration par voie intramammaire pendant la période sèche de l'animal, comprenant un agent antibactérien sous la forme de particules non-enduites dont au moins 65% en poids ont une dimension comprise dans la gamme de 0 à 5 µm, au moins 94% en poids ont une dimension comprise dans la gamme de 0 à 13 µm et moins de 55% en poids ont une dimension supérieure à 3 µm, en suspension dans un véhicule huileux hydrophobe qui comprend une huile minérale et un agent gélifiant, dans lequel le mélange obtenu par mélange de l'agent antibactérien avec le véhicule est traité dans un broyeur à billes.

2. Procédé suivant la revendication 1, dans lequel l'huile minérale est une paraffine liquide.

3. Procédé suivant la revendication 1 ou la revendication 2, dans lequel l'agent antibactérien est une pénicilline ou un sel ou un ester non toxique de celle-ci.

4. Procédé suivant l'une quelconque des revendications précédentes, dans lequel l'agent antibactérien comprend de la benzathine cloxacilline et/ou de l'ampicilline.

5. Procédé suivant la revendication 4, dans lequel la composition vétérinaire est une dose unitaire contenant 600 mg de benzathine cloxacilline.
